# EUROPÄISCHE PATENTSCHRIFT

(11) **EP 0 657 437 B1**
(45) Veröffentlichungstag und Bekanntmachung des Hinweises auf die Patenterteilung: **27.08.1997**
(21) Anmeldenummer: 94118675.1
(22) Anmeldetag: 28.11.1994
(51) Int. Cl.: C07D 249/12

(54) **Verfahren und neue Zwischenprodukte zur Herstellung von Triazolinonen**
Process and novel intermediates for the preparation of triazolinones
Procédé et nouveaux intermédiaires pour la préparation de triazolinones

(30) Priorität: 10.12.1993 DE 4342190
(43) Veröffentlichungstag der Anmeldung: 14.06.1995
(73) Patentinhaber: BAYER AG, 51368 Leverkusen (DE)
(72) Erfinder: Haas, Wilhelm Dr., D-50259 Pulheim (DE); Linker, Karl-Heinz, D-51377 Leverkusen (DE); Findeisen, Kurt Prof. Dr., D-51375 Leverkusen (DE)

(56) Entgegenhaltungen:
- CH-A- 502 375
- US-A- 3 780 052
- 'METHODEN DER ORGANISCHEN CHEMIE (HOUBEN-WEYL)' 1983 , GEORG THIEME VERLAG , SUTTGART UND NEW-YORK * Band E4, Seiten 349, 384 und 385 *
- JOURNAL OF MEDICINAL CHEMISTRY, Bd.14, Nr.3, März 1971, WASHINGTON US Seiten 260 - 262 M.Y. MHASALKAR ET AL. 'Further studies in substituted 4H-1,2,4-triazoles for possible hypoglycemic activity'
- 'COMPREHENSIVE HETEROCYCLIC CHEMISTRY (KATRITZKY)' 1984 , PERGAMON PRESS , OXFORD * Band 5, Teil 4A, Seiten 758, 759 *
- C. TEMPLE JR. 'THE CHEMISTRY OF HETEROCYCLIC COMPOUNDS (WEISSBERGER), TRIAZOLES 1,2,4' 1981 , JOHN WILEY & SONS , NEW YORK * Seiten 261, 262, 286 und 287 *
- IL FARMACO, EDIZIONE SCIENTIFICA, Bd.36, Nr.3, 1981, PAVIA IT Seiten 181 - 196 G. MAZZONE ET AL. 'Sintesi di 1-aroil-4H(R)-tiosemicarbazidi, dei correspondenti 5-aril-4H(R)-1,2,4-triazolin-3-tioni e di alcuni derivati di interesse farmaceutico'

## Beschreibung

Die Erfindung betrifft ein neues Verfahren und neue Zwischenprodukte zur Herstellung von weitgehend bekannten Triazolinonen, welche als Zwischenprodukte zur Herstellung von Herbiziden und Insektiziden verwendet werden können.

Es ist bekannt, daß man bestimmte substituierte Triazolinone, wie z.B. die Verbindung 4-Methyl-5-trifluormethyl-2,4-dihydro-3H-1,2,4-triazol-3-on 1,2,4-triazol-3-on erhält, wenn man entsprechende Triazolinthione, wie z.B. die Verbindung 4-Methyl-5-trifluormethyl-2,4-dihydro-3H-1,2,4-triazol-3-thion, zunächst mit einem Alkylierungsmittel, wie z.B. Methyliodid, in Gegenwart eines Säurebindemittels, wie z.B. Natriummethylat umsetzt, das hierbei gebildete Alkylthiotriazolderivat auf übliche Weise isoliert, dann mit Hydrogenperoxid in Gegenwart von Essigsäure erhitzt, nach Abkühlen neutralisiert und auf übliche Weise aufarbeitet (vgl. US-P 3780052 - Example 2).

Weiter ist bekannt, daß die oben genannte Verbindung 4-Methyl-5-trifluormethyl-2,4-dihydro-3H-1,2,4-triazol-3-on auch durch Erhitzen von 1-Trifluoracetyl-4-methyl-semicarbazid auf 160°C bis 180°C, anschließende Extraktion mit Essigsäureethylester und Säulenchromatographie erhalten werden kann (vgl. US-P 3780052 - Example 3).

In Houben-Weyl, Methoden der Organischen Chemie, Band E4, 1983, S. 349 und 384, 385 wird ganz allgemein die Überführung von Thioharnstoffen in Harnstoffe und der Schwefel-Sauerstoff-Austausch in cyclischen Thioharnstoffen beschrieben.

Die CH-A-502 375 beschreibt u.a. die Entschwefelung einer 5-(Nitro-2-furfuryliden-amino)mercapto-triazolverbindung.

Comprehensive Heterocyclic Chemistry (Kartritzky) Band 5, Teil 4A, 1984, S. 758, 759 beschreibt in allgemeiner Form die elektrolytische Oxidation von Thionen zu den entsprechenden Sulfonsäuren oder entschwefelten Verbindungen.

The Chemistry of heterocyclic compounds (Weissberger), Triazoles 1,2,4 (1981), S. 261, 262, 286 und 287 beschreibt die Oxidation von Triazolinthion mit Kaliumpermanganat oder Chlor zum Triazolsulfonsäurechlorid oder zur Triazol sulfonsäure.

II Farmaco, Ed. Sc. Vol. 36, 1981, Seiten 181-196 beschreibt die Reaktion von Mercaptotriazol mit Kaliumpermanganat in Natronlauge über die entsprechende Sulfonsäure zum Triazolinon.

J. Med. Chem. Band 14, 1971, Seiten 260-262 beschreibt den Ersatz einer Mercaptogruppe in 3 Positionen eines substituierten 4H-1,2,4-Triazols durch 3-Alkyl, SO₂-Alkyl, Wasserstoff oder OH.

Bei den beiden angegebenen Synthesemethoden sind jedoch Ausbeute und Qualität der erhaltenen Produkte sehr unbefriedigend.

Gegenstand der vorliegenden Anmeldung ist ein neues Verfahren zur Herstellung von Triazolinonen der allgemeinen Formel (I) 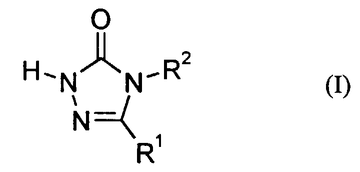 in welcher
- R¹: für gegebenenfalls durch Halogen, Cyano oder C₁-C₄-Akoxy substituiertes Alkyl mit 1 bis 6 Kohlenstoffatomen oder für gegebenenfalls durch Halogen, Cyano oder C₁-C₄-Alkyl substituiertes Cycloalkyl mit 3 bis 6 Kohlenstoffatomen steht und
- R²: für Amino, für einen jeweils gegebenenfalls durch Halogen, Cyano oder C₁-C₄-Alkoxy substituierten Rest der Reihe Alkyl, Alkenyl, Alkinyl, Alkoxy, Alkylamino oder Dialkylamino mit jeweils bis zu 6 Kohlenstoffatomen in den Alkyl-, Alkenyl- oder Alkinylgruppen oder für einen jeweils gegebenenfalls durch Halogen, Cyano, C₁-C₄-Alkyl, C₁-C₄-Alkoxy oder C₁-C₄-Alkoxycarbonyl substituierten Rest der Reihe C₃-C₆-Cycloalkyl, C₃-C₆-Cycloalkyl-C₁-C₂-alkyl oder Phenyl steht,
dadurch gekennzeichnet, daß man
Triazolinthione der allgemeinen Formel (II) 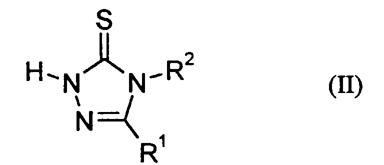 in welcher
- R¹ und R²: die oben angegebenen Bedeutungen haben,
mit einem Oxidationsmittel gegebenenfalls in Gegenwart eines Reaktionshilfsmittels und gegebenenfalls in Gegenwart eines Verdünnungsmittels bei Temperaturen zwischen 0°C und 100°C umsetzt, und die hierbei ("in der ersten Stufe des erfindungsgemäßen Verfahrens") synthetisierten Triazolsulfonsäuren bzw. deren Salze der allgemeinen Formel (m) 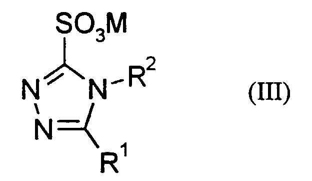 in welcher
- R¹ und R²: die oben angegebenen Bedeutungen haben und
- M: für Wasserstoff oder ein Metalläquivalent steht,
- gegebenenfalls nach Zwischenisolierung - ("in der zweiten Stufe des erfindungsgemäßen Verfahrens") mit Wasser in Gegenwart einer Säure bei Temperaturen zwischen 20°C und 120°C umsetzt.

Überraschenderweise können nach dem erfindungsgemäßen Verfahren die Triazolinone der allgemeinen Formel (I) auf einfache Weise in hohen Ausbeuten, welche im Vergleich mit dem Stand der Technik erheblich verbessert sind, und in hoher Reinheit erhalten werden.

Das erfindungsgemäße Verfahren stellt somit eine wertvolle Bereicherung des Standes der Technik dar.

Nach dem erfindungsgemäßen Verfahren werden vorzugsweise Verbindungen der Formel (I) hergestellt, in welcher
- R¹: für jeweils gegebenenfalls durch Fluor, Chlor, Brom, Cyano, Methoxy oder Ethoxy substituiertes Methyl, Ethyl, n- oder i-Propyl, n-, i- oder s-Butyl oder für jeweils gegebenenfalls durch Fluor, Chlor, Brom, Cyano oder Methyl substituiertes Cyclopropyl, Cyclopropylmethyl, Cyclobutyl, Cyclopentyl oder Cyclohexyl steht, und
- R²: für Amino, für einen jeweils gegebenenfalls durch Fluor, Chlor, Cyano, Methoxy oder Ethoxy substituierten Rest der Reihe Methyl, Ethyl, n- oder i-Propyl, n-, i- oder s-Butyl, Allyl, Propargyl, Methoxy, Ethoxy, n- oder i-Propoxy, n-, i- oder s-Butoxy, Methylamino, Ethylamino, n- oder i-Propylamino, n-, i- oder s-Butylamino, Dimethylamino oder Diethylamino oder für einen jeweils gegebenenfalls durch Fluor, Chlor, Brom, Cyano, Methyl, Ethyl, n- oder i-Propyl, Methoxy-carbonyl oder Ethoxycarbonyl substituierten Rest der Reihe Cyclopropyl, Cyclobutyl, Cyclopentyl oder Cyclohexyl oder Phenyl steht.

Die bei den Restedefinitionen genannten Kohlenwasserstoffreste, wie Alkyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Eine ganz besonders bevorzugte Gruppe von Verbindungen, welche nach dem erfindungsgemäßen Verfahren hergestellt werden können, sind die Verbindungen der Formel (I), in welcher
- R¹: für einfach, zweifach, dreifach, vierfach, fünffach, sechsfach oder siebenfach durch Fluor und/oder Chlor substituiertes Methyl, Ethyl, n- oder i-Propyl, Cyclopropyl oder Cyclopropylmethyl steht und
- R²: für Methyl, Ethyl, n- oder i-Propyl, Methoxy, Ethoxy, Methylamino, Ethylamino, Dimethylamino, Cyclopropyl, Phenyl oder Tolyl steht.

Die oben aufgeführten allgemeinen oder in Vorzugsbereichen angegebenen Restedefinitionen gelten sowohl für die Endprodukte der Formel (I) als auch entsprechend für die jeweils zurHerstellung benötigten Ausgangsstoffe bzw. Zwischenprodukte.
Diese Restedefinitionen können untereinander, also auch zwischen den angegebenen Bereichen bevorzugter Verbindungen, beliebig kombiniert werden.

Verwendet man beispielsweise 5-Difluormethyl-4-methoxy-2,4-dihydro-3H-1,2,4-triazol-3-thion und Wasserstoffperoxid als Ausgangsstoffe, so kann der Reaktionsablauf beim erfindungsgemäßen Verfahren durch das folgende Formelschema skizziert werden: 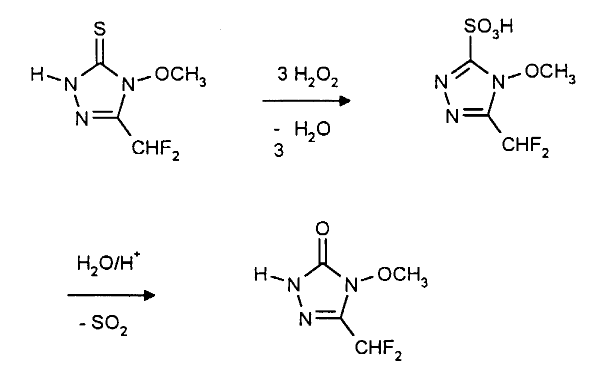 Die beim erfindungsgemäßen Verfahren zur Herstellung der Verbindungen der allgemeinen Formel (I) als Ausgangsstoffe zu verwendenden Triazolinthione sind durch die Formel (II) allgemein definiert. In der Formel (II) haben R¹ und R² vorzugsweise bzw. insbesondere diejenige Bedeutung, die bereits oben im Zusammenhang mit der Beschreibung der Verbindungen der Formel (I) als bevorzugt bzw. als insbesondere bevorzugt für R¹ und R² angegeben wurde.

Die Triazolinthione der allgemeinen Formel (II) sind bekannt und/oder können nach an sich bekannten Verfahren hergestellt werden (vgl. US-P 3719686 und US-P 3780052).

Man erhält die Triazolinthione der allgemeinen Formel (II), wenn man Carbonsäuren der allgemeinen Formel (IV)

R¹-CO-X (IV)

in welcher
- R¹: die oben angegebene Bedeutung hat und
- X: für Hydroxy oder Halogen steht,
mit Alkylthiosemicarbaziden der allgemeinen Formel (V)

R²-NH-CS-NH-NH₂ (V)

in welcher
- R²: die oben angegebene Bedeutung hat,
bei Temperaturen zwischen 0°C und 100°C umsetzt (vgl. die Herstellungsbeispiele).

Die bei der Durchführung des erfindungsgemäßen Verfahrens intermediär synthetisierten Triazolsulfonsäuren und deren Salze der allgemeinen Formel (III) sind noch nicht aus der Literatur bekannt; sie sind als neue Stoffe Gegenstand der vorliegenden Anmeldung.

In der Formel (III) haben R¹ und R² vorzugsweise bzw. insbesondere diejenige Bedeutung, die bereits oben im Zusammenhang mit der Beschreibung der Verbindungen der Formel (I) als bevorzugt bzw. als insbesondere bevorzugt für R¹ und R² genannt wurde; M steht vorzugsweise für ein Alkalimetall- oder ein Erdalkalimetall-äquivalent, insbesondere für Natrium oder Kalium.

Das erfindungsgemäße Verfahren wird unter Einsatz eines Oxidationsmittels durchgeführt. Geeignete Oxidationsmittel sind dafür beispielsweise Sauerstoff, Ozon, Hydrogenperoxid, Chlor, Chlorlauge, Natrium- oder Kalium-permanganat, Salpetersäure, Natrium- oder Kalium-dichromat, Natrium- oder Kalium-peroxodisulfat, Natrium- oder Kalium-perborat, Perameisensäure, Peressigsäure, Perpropionsäure sowie gegebenenfalls halogenierte Perbenzoesäuren. Hydrogenperoxid (Wasserstoffperoxid) wird hierbei als Oxidationsmittel besonders bevorzugt.

Geeignete Reaktionshilfsmittel - insbesondere bei Einsatz von Hydrogenperoxid - sind hierbei vor allem Salze von Metallen der IV., V. und VI. Nebengruppe des Periodensystems der Elemente. Als Beispiele hierfür seien Natrium(meta)vanadat, Natriummolybdat und Natriumwolframat genannt.

Geeignete Reaktionshilfsmittel sind weiterhin die gewöhnlich als Säureakzeptoren verwendeten Chemikalien. Als solche kommen alle üblichen anorganischen oder organischen Basen infrage. Hierzu gehören beispielsweise Erdalkali- oder Alkalimetall-hydroxide, -alkoholate, -acetate, -carbonate oder -hydrogencarbonate, wie beispielsweise Natriumhydroxid, Kaliumhydroxid, Natrium-methylat, Natriumethylat, Kalium-tert.-butylat, Natriumacetat, Kaliumacetat, Calciumacetat, Natriumcarbonat, Kaliumcarbonat, Kaliumhydrogencarbonat oder Natriumhydrogencarbonat.
Insbesondere Natriumhydroxid und Kaliumhydroxid werden als Reaktionshilfsmittel bei der ersten Stufe des erfindungsgemäßen Verfahrens bevorzugt.

Das erfindungsgemäße Verfahren wird vorzugsweise unter Verwendung eines Verdünnungsmittels durchgeführt. Geeignete Verdünnungsmittel sind hierbei insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methyl-formanilid, N-Methyl-pyrrolidon oder Hexamethylphosphorsäuretriamid; Carbonsäuren wie Ameisensäure, Essigsäure oder Propionsäure, Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser. Insbesondere Wasser wird bei der ersten Stufe des erfindungsgemäßen Verfahrens als Verdünnungsmittel bevorzugt.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 0°C und 100°C, vorzugsweise bei Temperaturen zwischen 20°C und 80°C, insbesondere bei Temperaturen zwischen 30°C und 60°C.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung der ersten Stufe des erfindungsgemäßen Verfahrens setzt man auf 1 Mol Triazolinthion der Formel (II) im allgemeinen zwischen 3 und 4 Mol, vorzugsweise zwischen 3,0 und 3,5 Moläquivalente eines Oxidationsmittels ein.

Die zweite Stufe des erfindungsgemäßen Verfahrens wird gegebenenfalls in Gegenwart einer Säure durchgeführt. Vorzugsweise werden hierbei anorganische oder organische Protonensäuren, wie z.B. Salzsäure, Schwefelsäure, Methansulfonsäure, Benzolsulfonsäure oder p-Toluolsulfonsäure eingesetzt.
Insbesondere Salzsäure wird bei der zweiten Stufe des erfindungsgemäßen Verfahrens eingesetzt.

Wasser dient in der zweiten Stufe des erfindungsgemäßen Verfahrens gleichzeitig als Reaktionskomponente und als Verdünnungsmittel. Es wird daher in hohem Überschuß eingesetzt. Je Mol Zwischenprodukt der Formel (III) werden im allgemeinen zwischen 0,5 und 5 Liter, vorzugsweise zwischen 1 und 3 Liter Wasser verwendet.

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen 20°C und 120°C, vorzugsweise bei Temperaturen zwischen 50°C und 110°C, insbesondere bei Temperaturen zwischen 80°C und 100°C.

In einer bevorzugten Ausführungsform des erfindungsgemäßen Verfahrens werden beide Stufen in getrennten Ansätzen durchgeführt.

Zur Durchführung der ersten Stufe wird vorzugsweise die Ausgangsverbindung der Formel (II) in wässriger Alkalilauge gelöst und dann das Oxidationsmittel langsam zudosiert. Das Reaktionsgemisch wird bis zum Ende der Umsetzung gerührt und das Zwischenprodukt der Formel (III) nach üblichen Methoden isoliert.

Beispielsweise wird überschüssiges Oxidationsmittel mit Natriumhydrogensulfit zersetzt, dann - beispielsweise mit Salzsäure - angesäuert und mit einem mit Wasser praktisch nicht mischbaren organischen Lösungsmittel, wie z.B. Essigsäureethylester, gewaschen. Nach Einengen der wäßrigen Lösung fällt das ZwischenproduktderFormel(III)kristallinanundkanndurchAbfiltrierenisoliertwerden.

Zur Durchführung der zweiten Stufe wird vorzugsweise das Zwischenprodukt der Formel (III) mit Wasser, das eine Säure enthält, verrührt und die Mischung bis zum Ende der Umsetzung erhitzt. Die Aufarbeitung kann auf übliche Weise durchgeführt werden. Beispielsweise wird mit einem mit Wasser praktisch nicht mischbaren organischen Lösungsmittel, wie z.B. Methylenchlorid mehrfach extrahiert; die vereinigten organischen Extraktionslösungen werden getrocknet und filtriert. Nach sorgfältigem Abdestillieren des Lösungsmittels vom Filtrat erhält man das Produkt der Formel (I) als Rückstand.

In einer weiteren bevorzugten Ausführungsform des erfindungsgemäßen Verfahrens werden beide Stufen in einem einzigen Ansatz, d.h. ohne Isolierung des Zwischenproduktes der Formel (III) durchgeführt.

Hierbei wird vorzugsweise das Triazolinthion der Formel (II) in wässriger Alkalilauge gelöst und dann das Oxidationsmittel langsam zudosiert. Das Reaktionsgemisch wird bis zum Ende der Umsetzung gerührt, dann überschüssiges Oxidationsmittel beispielsweise mit Natriumbisulfit vernichtet, anschließend eine Säure, wie z.B. konz. Salzsäure zum Reaktionsgemisch gegeben und die Mischung auf die für die zweite Stufe erforderliche Temperatur aufgeheizt. Nach Ende der Umsetzung wird wie oben für die zweite Stufe beispielhaft beschrieben aufgearbeitet.

Die nach dem erfindungsgemäßen Verfahren herzustellenden Triazolinone der Formel (I) können als Zwischenprodukte zur Herstellung von landwirtschaftlich nutzbaren Wirkstoffen verwendet werden (vgl. US-P 3780052, US-P 3780053, US-P 3780054 und EP-A 341489).

### Herstellungsbeispiele:

### Beispiele für die erste Stufe:

### Beispiel 1

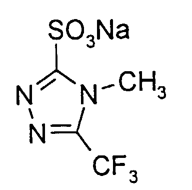

25 g (0,14 Mol) 4-Methyl-5-trifluormethyl-2,4-dihydro-3H-1,2,4-triazol-3-thion werden in 250 ml 2,5-molarer Natronlauge gelöst und innerhalb von 60 Minuten bei einer Reaktionstemperatur von 40°C bis 45°C (Außenkühlung) mit 46,5 g einer 35%igen Hydrogenperoxid-Lösung (0,48 Mol H₂O₂) versetzt. Die Mischung wird 6 Stunden bei 40°C gerührt; dann wird überschüssiges Oxidationsmittel mit Natriumhydrogensulfit zersetzt und die Lösung durch Zugabe von 18-molarer Salzsäure auf pH=2 angesäuert. Anschließend wird mit Essigsäureethylester gewaschen und die wässrige Lösung dann im Wasserstrahlvakuum bis auf etwa 200 ml eingeengt. Der beim Abkühlen anfallende Feststoff wird durch Abfiltrieren isoliert. Man erhält 25 g (73% der Theorie) 4-Methyl-5-trifluormethyl-4H-1,2,4-triazol-3-sulfonsäure-Natriumsalz.
Schmelzpunkt >230°C;
¹H-NMR (DMSO-D₆, δ): 3,92 ppm.

### Beispiel 2

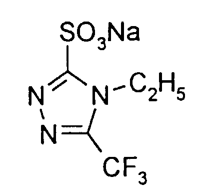

9,9 g (0,05 Mol) 4-Ethyl-5-trifluormethyl-2,4-dihydro-3H-1,2,4-triazol-3-thion werden in 50 ml 2,5 molarer Natronlauge vorgelegt. Bei maximal 40°C - 50°C (Kühlung) tropft man innerhalb 20 Minuten 17 ml (0,165 Mol H₂O₂) 30%ige Wasserstoffperoxid-Lösung zu, rührt 6 Stunden bei 40°C nach, beseitigt den Überschuß an Wasserstoffperoxid durch Zugabe von Natriumhydrogensulfit, säuert mit konzentrierter Salzsäure auf pH 2 an, rührt 1 Stunde bei Raumtemperatur und extrahiert mehrfach mit Essigester. Die wäßrige Phase wird zu 2/3 eingeengt und das ausgefallene Produkt abgesaugt.

Man erhält 9,1 g (74,3% der Theorie) 4-Ethyl-5-trifluormethyl-4H-1,2,4-triazol-3-sulfonsäure-Na-Salz vom Schmelzpunkt >250°C.

### Beispiele für die zweite Stufe:

### Beispiel 3

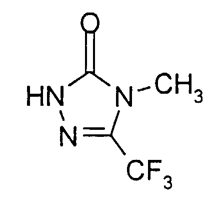

20 g (0,08 Mol) 4-Methyl-5-trifluormethyl-4H-1,2,4-triazol-3-sulfonsäure-Natriumsalz werden in 200 ml 10%iger Salzsäure suspendiert und die Mischung 12 Stunden unter Rückfluß erhitzt. Es entsteht eine klare Lösung, die nach dem Erkalten mehrfach mit Dichlormethan extrahiert wird. Die vereinigten organischen Extraktionslösungen werden mit Natriumsulfat getrocknet und filtriert. Vom Filtrat wird das Lösungsmittel im Wasserstrahlvakuum sorgfältig abdestilliert.Man erhält 8,2 g (62% der Theorie) 4-Methyl-5-trifluormethyl-2,4-dihydro-3H-1,2,4-triazol-3-on als festen Rückstand vom Schmelzpunkt 64°C.

### Beispiele für das Gesamtverfahren:

### Beispiel 4

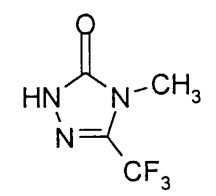

18,3 g (0,1 Mol) 4-Methyl-5-trifluormethyl-2,4-dihydro-3H-1,2,4-triazol-3-thion werden in 100 ml 2,5-molarer Natronlauge vorgelegt. Bei 40°C bis 50°C wird eine Hydrogenperoxid-Lösung (34 ml 30%iges Hydrogenperoxid: 0,33 Mol H₂O₂) langsam zugetropft und die Mischung noch 2 Stunden bei 50°C nachgerührt. Dann wird überschüssiges Oxidationsmittel mit Natriumbisulfit beseitigt und anschließend die Mischung mit konzentrierter Salzsäure angesäuert. Das Reaktionsgemisch wird dann 15 Stunden unter Rückfluß erhitzt, anschließend in Eiswasser eingerührt und mit Methylenchlorid extrahiert. Die Extraktionslösung wird mit Natriumsulfat getrocknet und filtriert. Vom Filtrat wird das Lösungsmittel im Wasserstrahlvakuum sorgfältig abdestilliert.Man erhält 11,3 g (68% der Theorie) 4-Methyl-5-trifluormethyl-2,4-dihydro-3H-1,2,4-triazol-3-on als festen Rückstand vom Schmelzpunkt 64°C.

### Beispiel 5

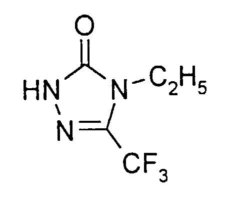

9,9 g (0,05 Mol) 4-Ethyl-5-trifluormethyl-2,4-dihydro-3H-1,2,4-triazol-3-thion und 50 ml 2,5 molarer Natronlauge werden vorgelegt. Bei einer maximalen Temperatur von 40°C bis 50°C (Kühlung) tropft man 17 ml (0,165 Mol) 30%ige Wasserstoffperoxid-Lösung innerhalb 20 Minuten zu, rührt 6 Stunden bei 40°C nach, beseitigt den Überschuß an Wasserstoffperoxid durch Zugabe von Natriumhydrogensulfit, säuert mit konzentrierter Salzsäure an und erhitzt für 10 Stunden zum Rückflußsieden. Nach dem Abkühlen gießt man auf Eiswasser, extrahiert mehrfach mit Dichlormethan, trocknet die vereinigten organischen Phasen über Natriumsulfat und verdampft das Lösungsmittel am Rotationsverdampfer.Man erhält 5,2 g (58% der Theorie) 4-Ethyl-5-trifluormethyl-2,4-dihydro-3H-1,2,4-triazol-3-on vom Schmelzpunkt 53°C.

Analog der Beispiele 3 bis 5 können beispielsweise auch die in der nachstehenden Tabelle 1 aufgeführten Verbindungen der Formel (I) hergestellt werden.

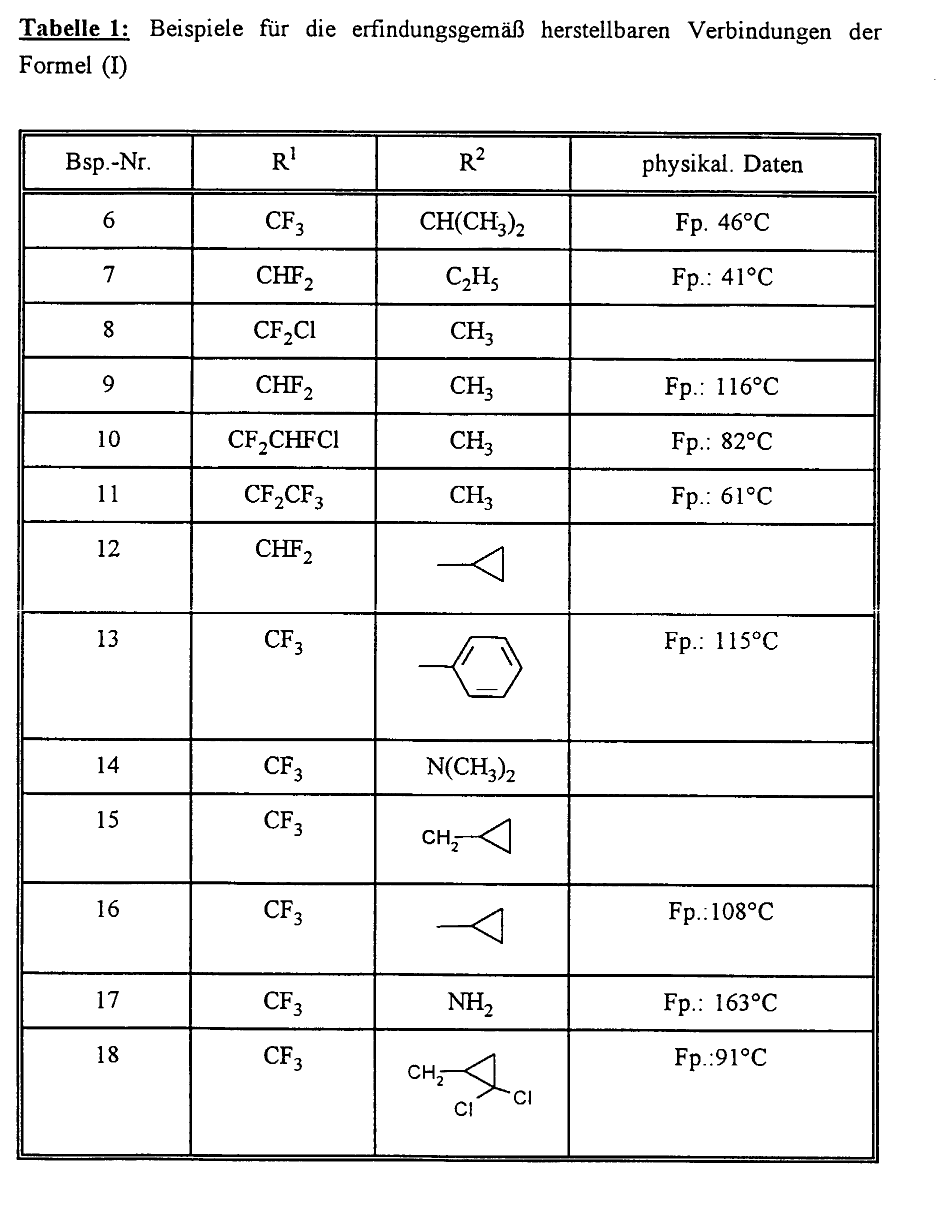

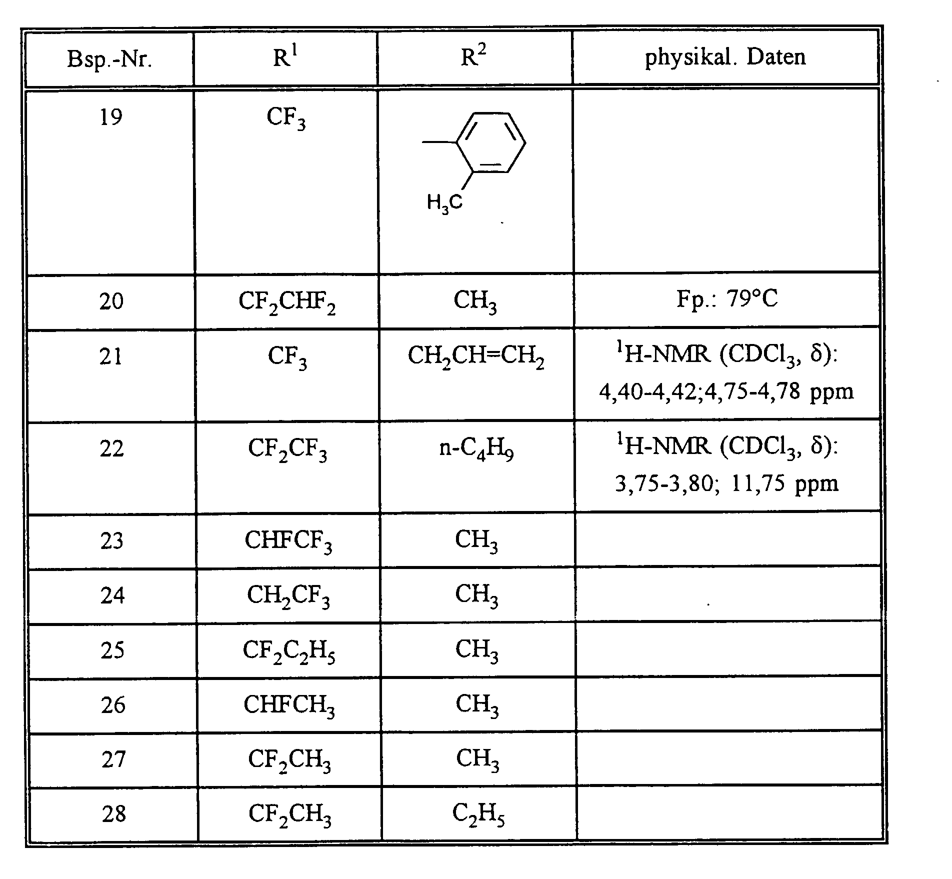

Analog der Beispiele 1 oder 2 können beispielsweise auch die in der nachstehenden Tabelle 2 aufgeführten Verbindungen der Formel (III) hergestellt werden.

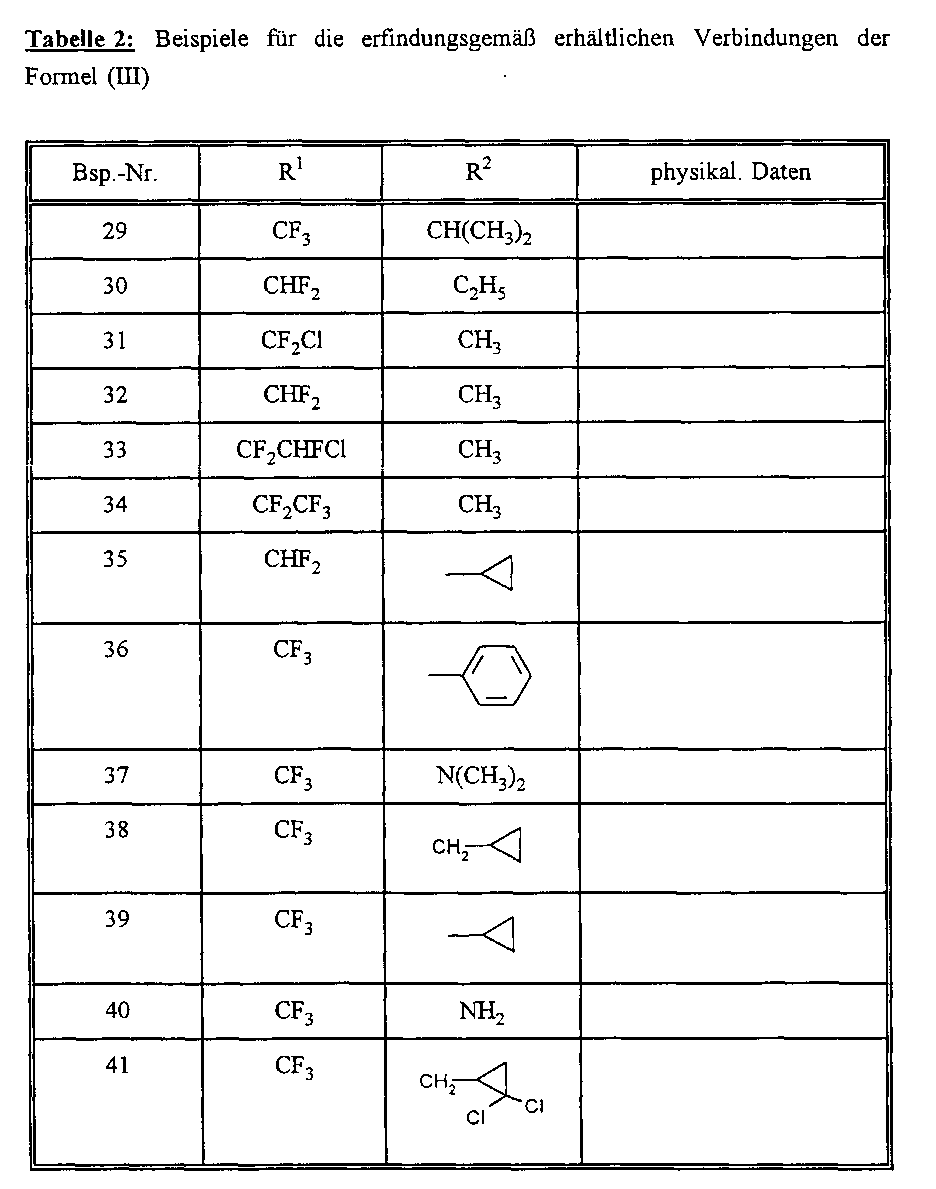

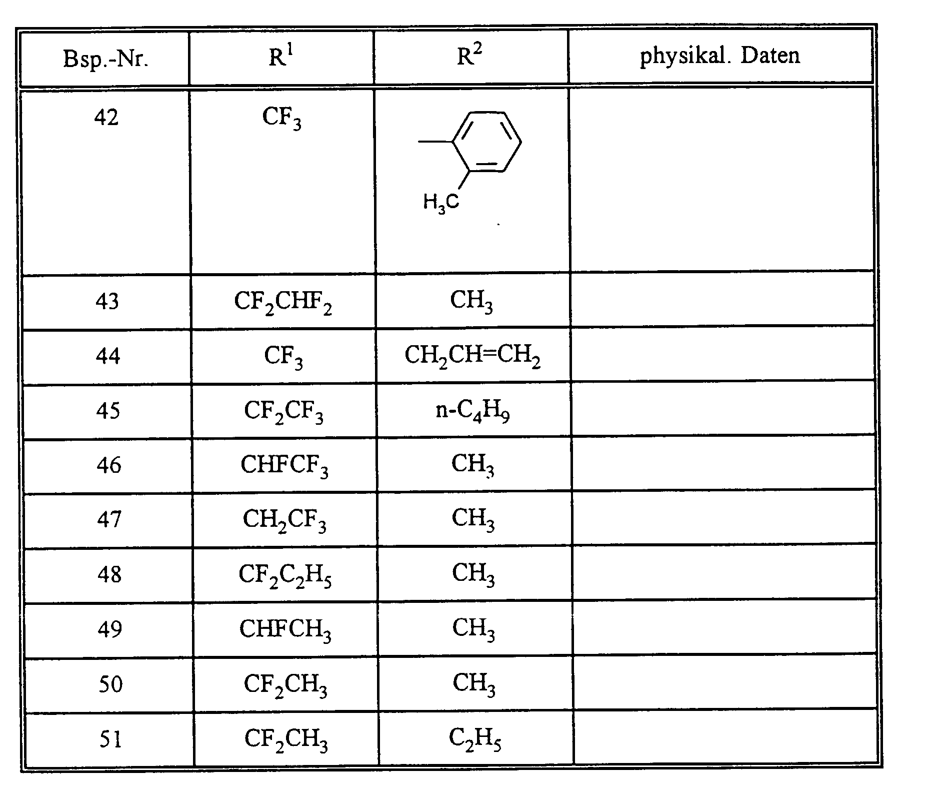

### Ausgangsstoffe der Formel (II):

### Beispiel (II-1)

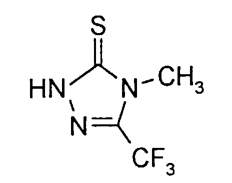

257 g (2,44 Mol) 4-Methyl-thiosemicarbazid werden zu 1,2 Liter Trifluoressigsäure gegeben und das Gemisch 16 Stunden unter Rückfluß erhitzt. Dann wird im Wasserstrahlvakuum eingeengt, der Rückstand mit Diethylether/Petrolether (Vol.: 5:100) verrieben und das kristallin anfallende Produkt durch Abfiltrieren isoliert.Man erhält 444,5 g (95,5% der Theorie) 4-Methyl-5-trifluormethyl-2,4-dihydro-3H-1,2,4-triazol-3-thion vom Schmelzpunkt 115°C.

## Patentansprüche

1. Verfahren zur Herstellung von Triazolinonen der allgemeinen Formel (I) 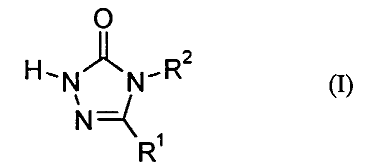 in welcher
R¹ für gegebenenfalls durch Halogen, Cyano oder C₁-C₄-Alkoxy substituiertes Alkyl mit 1 bis 6 Kohlenstoffatomen oder für gegebenenfalls durch Halogen, Cyano oder C₁-C₄-Alkyl substituiertes Cycloalkyl mit 3 bis 6 Kohlenstoffatomen steht und
R² für Amino, für einen jeweils gegebenenfalls durch Halogen, Cyano oder C₁-C₄-Alkoxy substituierten Rest der Reihe Alkyl, Alkenyl, Alkinyl, Alkoxy, Alkylamino oder Dialkylamino mit jeweils bis zu 6 Kohlenstoffatomen in den Alkyl-, Alkenyl- oder Alkinylgruppen oder für einen jeweils gegebenenfalls durch Halogen, Cyano, C₁-C₄-Alkyl, C₁-C₄-Alkoxy oder C₁-C₄-Alkoxycarbonyl substituierten Rest der Reihe C₃-C₆-Cycloalkyl, C3-C6-Cycloalkyl-C₁-C₂-alkyl oder Phenyl steht,
dadurch gekennzeichnet, daß man
Triazolinthione der allgemeinen Formel (II) 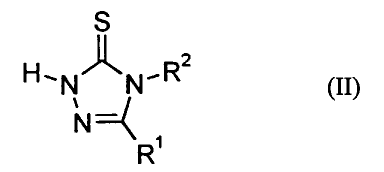 in welcher
R¹ und R² die oben angegebenen Bedeutungen haben,
mit einem Oxidationsmittel gegebenenfalls in Gegenwart eines Reaktionshilfsmittels und gegebenenfalls in Gegenwart eines Verdünnungsmittels bei Temperaturen zwischen 0°C und 100°C umsetzt,
und die hierbei ("in der ersten Stufe des erfindungsgemäßen Verfahrens") synthetisierten Triazolsulfonsäuren bzw. deren Salze der allgemeinen Formel (III) 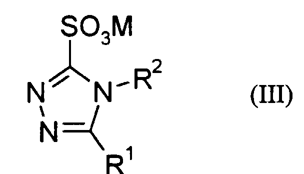 in welcher
R¹ und R² die oben angegebenen Bedeutungen haben und
M für Wasserstoff oder ein Metalläquivalent steht,
- gegebenenfalls nach Zwischenisolierung - ("in der zweiten Stufe des erfindungsgemäßen Verfahrens") mit Wasser in Gegenwart einer Säure bei Temperaturen zwischen 20°C und 120°C umsetzt.

2. Verfahren zur Herstellung von Triazolinonen der allgemeinen Formel (I) gemäß Anspruch 1, dadurch gekennzeichnet, daß
R¹ für jeweils gegebenenfalls durch Fluor, Chlor, Brom, Cyano, Methoxy oder Ethoxy substituiertes Methyl, Ethyl, n- oder i-Propyl, n-, i- oder s-Butyl oder für jeweils gegebenenfalls durch Fluor, Chlor, Brom, Cyano oder Methyl substituiertes Cyclopropyl, Cyclopropylmethyl, Cyclobutyl, Cyclopentyl oder Cyclohexyl steht und
R² für Amino, für einen jeweils gegebenenfalls durch Fluor, Chlor, Cyano, Methoxy oder Ethoxy substituierten Rest der Reihe Methyl, Ethyl, n- oder i-Propyl, n-, i- oder s-Butyl, Allyl, Propargyl, Methoxy, Ethoxy, n- oder i-Propoxy, n-, i- oder s-Butoxy, Methylamino, Ethylamino, n- oder i-Propylamino, n-, i- oder s-Butylamino, Dimethylamino oder Diethylamino oder für einen jeweils gegebenenfalls durch Fluor, Chlor, Brom, Cyano, Methyl, Ethyl, n- oder i-Propyl, Methoxycarbonyl oder Ethoxycarbonyl substituierten Rest der Reihe Cyclopropyl, Cyclobutyl, Cyclopentyl oder Cyclohexyl oder Phenyl steht.

3. Verfahren zur Herstellung von Triazolinonen der allgemeinen Formel (I) gemäß Anspruch 1, dadurch gekennzeichnet, daß
R¹ für einfach, zweifach, dreifach, vierfach, fünffach, sechsfach oder siebenfach durch Fluor und/oder Chlor substituiertes Methyl, Ethyl, n- oder i-Propyl, Cyclopropyl oder Cyclopropylmethyl steht und
R² für Methyl, Ethyl, n- oder i-Propyl, Methoxy, Ethoxy, Methylamino, Ethylamino, Dimethylamino, Cyclopropyl, Phenyl oder Tolyl steht.

4. Verfahren gemäß Anspruch 1, dadurch gekennzeichnet, daß man die Umsetzung in der ersten Phase des Verfahrens bei Temperaturen zwischen 20°C und 80°C und in der zweiten Phase des Verfahrens bei Temperaturen zwischen 50°C und 110°C durchführt.

5. Verfahren gemäß Anspruch 1, dadurch gekennzeichnet, daß man bei der Umsetzung in der ersten Phase des Verfahrens pro Mol Triazolinthion der Formel (II) 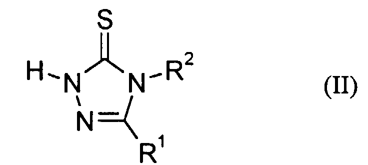 in welcher R¹ und R² die in Anspruch 1 genannten Bedeutungen haben, zwischen 3 und 4 Mol eines Oxidationsmittels einsetzt.

6. Verfahren gemäß Anspruch 1, dadurch gekennzeichnet, daß man das Verfahren unter Einsatz geeigneter Reaktionshilfsmittel durchführt.

7. Triazolsulfonsäuren bzw. deren Salze der allgemeinen Formel (III) 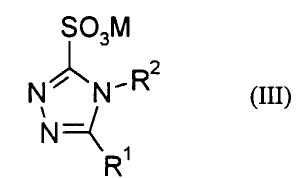 dadurch gekennzeichnet, daß
R¹ und R² die in Anspruch 1 angegebenen Bedeutungen haben und
M für Wasserstoff oder für ein Alkalimetall- oder ein Erdalkalimetalläquivalent steht.

8. Triazolsulfonsäuren bzw. deren Salze der allgemeinen Formel (III), gemäß Anspruch 7, dadurch gekennzeichnet, daß
R¹ und R² die in Anspruch 2 angegebenen Bedeutungen haben und
M für Wasserstoff, Natrium oder Kalium steht.

## Claims

1. Process for the preparation of triazolinones of the general formula (I) 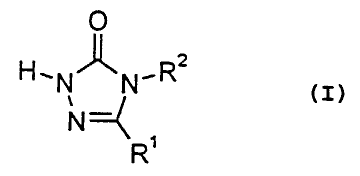 in which
R¹ represents alkyl having 1 to 6 carbon atoms which is optionally substituted by halogen, cyano or C₁-C₄-alkoxy, or represents cycloalkyl having 3 to 6 carbon atoms which is optionally substituted by halogen, cyano or C₁-C₄-alkyl, and
R² represents amino, a radical from the series comprising alkyl, alkenyl, alkinyl, alkoxy, alkylamino or dialkylamino, each of which has up to 6 carbon atoms in the alkyl, alkenyl or alkinyl groups and each of which is optionally substituted by halogen, cyano or C₁-C₄-alkoxy, or represents a radical from the series comprising C₃-C₆-cycloalkyl, C₃-C₆-cycloalkyl-C₁-C₂-alkyl or phenyl, each of which is optionally substituted by halogen, cyano, C₁-C₄-alkyl, C₁-C₄-alkoxy or C₁-C₄-alkoxycarbonyl,
characterized in that
triazolinethiones of the general formula (II) 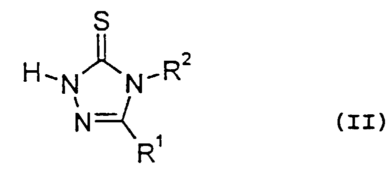 in which
R¹ and R² have the abovementioned meanings,
are reacted with an oxidant, if appropriate in the presence of a reaction auxiliary and if appropriate in the presence of a diluent, at temperatures between 0°C and 100°C,
and the synthesized triazolesulphonic acids or the salts thereof of the general formula (III) 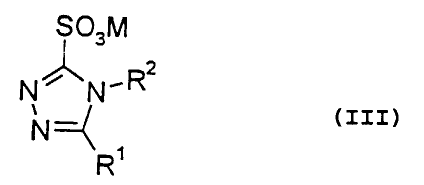 in which
R¹ and R² have the abovementioned meanings and
M represents hydrogen or a metal equivalent,
which have been obtained here ("in the first step of the process according to the invention") are reacted with water in the presence of an acid, at temperatures between 20°C and 120°C, if appropriate after an intermediate isolation step ("in the second step of the process according to the invention").

2. Process for the preparation of triazolinones of the general formula (I) according to Claim 1, characterized in that
R¹ represents methyl, ethyl, n- or i-propyl, or n-, i- or s-butyl, each of which is optionally substituted by fluorine, chlorine, bromine, cyano, methoxy or ethoxy, or represents cyclopropyl, cyclopropylmethyl, cyclobutyl, cyclopentyl or cyclohexyl, each of which is optionally substituted by fluorine, chlorine, bromine, cyano or methyl, and
R² represents amino, a radical from the series comprising methyl, ethyl, n- or i-propyl, n-, i- or s-butyl, allyl, propargyl, methoxy, ethoxy, n- or i-propoxy, n-, i- or s-butoxy, methylamino, ethylamino, n- or i-propylamino, n-, i- or s-butylamino, dimethylamino or diethylamino, each of which is optionally substituted by fluorine, chlorine, cyano, methoxy or ethoxy, or represents a radical from the series comprising cyclopropyl, cyclobutyl, cyclopentyl or cyclohexyl or phenyl, each of which is optionally substituted by fluorine, chlorine, bromine, cyano, methyl, ethyl, n- or i-propyl, methoxycarbonyl or ethoxycarbonyl.

3. Process for the preparation of triazolinones of the general formula (I) according to Claim 1, characterized in that
R¹ represents methyl, ethyl, n- or i-propyl, cyclopropyl or cyclopropylmethyl, each of which is mono-, di-, tri-, tetra-, penta-, hexa- or heptasubstituted by fluorine and/or chlorine and
R² represents methyl, ethyl, n- or i-propyl, methoxy, ethoxy, methylamino, ethylamino, dimethylamino, cyclopropyl, phenyl or tolyl.

4. Process according to Claim 1, characterized in that the reaction is carried out at temperatures between 20°C and 80°C in the first phase of the process and at temperatures between 50°C and 110°C in the second phase of the process.

5. Process according to Claim 1, characterized in that, in the reaction in the first phase of the process, between 3 and 4 mole of an oxidant are employed per mole of triazolinethione of the formula (II) 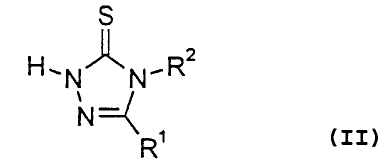 in which R¹ and R² have the meanings given in Claim 1.

6. Process according to Claim 1, characterized in that the process is carried out using suitable reaction auxiliaries.

7. Triazolesulphonic acids or their salts of the general formula (III) 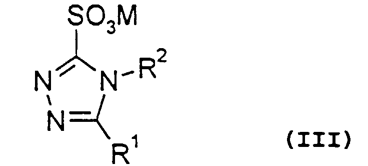 characterized in that
R¹ and R² have the meanings mentioned in Claim 1 and
M represents hydrogen or an alkali metal equivalent or alkaline earth metal equivalent.

8. Triazolesulphonic acids or their salts of the general formula (III) according to Claim 7, characterized in that
R¹ and R² have the meanings mentioned in Claim 2 and
M represents hydrogen, sodium or potassium.

## Revendications

1. Procédé pour la préparation de triazolinones répondant à la formule qénérale (I) 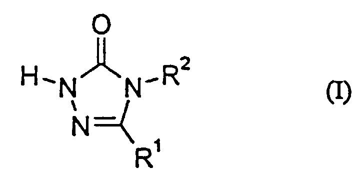 dans laquelle
R¹ représente un groupe alkyle contenant de 1 à 6 atomes de carbone portant éventuellement un ou plusieurs substituants identiques ou différents halogéno, cyano ou alcoxy en C₁-C₄; ou encore un groupe cycloalkyle contenant de 3 à 6 atomes de carbone portant éventuellement un ou plusieurs substituants identiques ou différents halogéno, cyano ou alkyle en C₁-C₄, et
R² représente un groupe amino; un radical choisi parmi la série comprenant un groupe alkyle, un groupe alcényle, un groupe alcynyle, un groupe alcoxy, un groupe alkylamino ou un groupe dialkylamino, chacun de ces groupes contenant jusqu'à 6 atomes de carbone dans les groupes alkyle, alcényle ou alcynyle, chacun de ces radicaux portant éventuellement un ou plusieurs substituants identiques ou différents halogéno, cyano ou alcoxy en C₁-C₄; ou encore un radical choisi parmi la série comprenant un groupe cycloalkyle en C₃-C₆, un groupe cycloalkyl(en C₃-C₆)alkyle en C₁-C₂ ou un groupe phényle, chacun de ces radicaux portant éventuellement un ou plusieurs substituants identiques ou différents halogéno, cyano, alkyle en C₁-C₄, alcoxy en C₁-C₄ ou alcoxy(en C₁-C₄)carbonyle,
caractérisé en ce qu'on fait réagir
des triazolinethiones répondant à la formule générale (II) 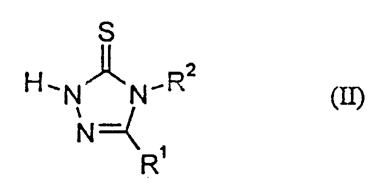 dans laquelle
R¹ et R² ont les significations indiquées ci-dessus,
avec un agent d'oxydation, éventuellement en présence d'un adjuvant réactionnel et éventuellement en présence d'un diluant, à des températures entre 0°C et 100°C,
et on fait réagir les acides triazolsulfoniques, respectivement leurs sels synthétisés en l'occurrence ("dans la première étape du procédé selon l'invention") répondant à la formule générale (III) 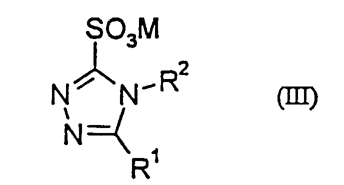 dans laquelle
R¹ et R² ont la signification indiquée ci-dessus, et
M représente un atome d'hydrogène ou un équivalent métallique,
- éventuellement après isolation intermédiaire - ("dans la seconde étape du procédé selon l'invention") avec de l'eau en présence d'un acide, à des températures entre 20°C et 120°C.

2. Procédé pour la préparation de triazolinones répondant à la formule générale (I) selon la revendication 1, caractérisé en ce que
R¹ représente un groupe méthyle, un groupe éthyle, un groupe n- ou i-propyle, un groupe n-, i- ou s-butyle, chacun de ces groupes portant éventuellement un ou plusieurs substituants identiques ou différents fluoro, chloro, bromo, cyano, méthoxy ou éthoxy; ou encore un groupe cyclopropyle, un groupe cyclopropylméthyle, un groupe cyclobutyle, un groupe cyclopentyle ou un groupe cyclohexyle, chacun de ces groupes portant éventuellement un ou plusieurs substituants identiques ou différents fluoro, chloro, bromo, cyano ou méthyle, et
R² représente un groupe amino; un radical choisi parmi la série comprenant un groupe méthyle, un groupe éthyle, un groupe n- ou i-propyle, un groupe n-, i- ou s-butyle, un groupe allyle, un groupe propargyle, un groupe méthoxy, un groupe éthoxy, un groupe n- ou i-propoxy, un groupe n-, i- ou s-butoxy, un groupe méthylamino, un groupe éthylamino, un groupe n- ou i-propylamino, un groupe n-, i-ou s-butylamino, un groupe diméthylamino ou un groupe diéthylamino, chacun de ces groupes portant éventuellement un ou plusieurs substituants identiques ou différents fluoro, chloro, cyano, méthoxy ou éthoxy; ou encore un radical choisi parmi la série comprenant un groupe cyclopropyle, un groupe cyclobutyle, un groupe cyclopentyle ou un groupe cyclohexyle ou encore un groupe phényle, chacun de ces groupes portant éventuellement un ou plusieurs substituants identiques ou différents fluoro, chloro, bromo, cyano, méthyle, éthyle, n- ou i-propyle, méthoxycarbonyle ou éthoxy-carbonyle.

3. Procédé pour la préparation de triazolinones répondant à la formule générale (I) selon la revendication 1, caractérisé en ce que
R¹ représente un groupe méthyle, un groupe éthyle, un groupe n- ou i-propyle, un groupe cyclopropyle ou un groupe cyclopropylméthyle portant un, deux, trois, quatre, cinq, six ou sept substituants identiques ou différents fluoro et/ou chloro, et
R² représente un groupe méthyle, un groupe éthyle, un groupe n- ou i-propyle, un groupe méthoxy, un groupe éthoxy, un groupe méthylamino, un groupe éthylamino, un groupe diméthylamino, un groupe cyclopropyle, un groupe phényle ou un groupe tolyle.

4. Procédé selon la revendication 1, caractérisé en ce qu'on effectue la mise en réaction dans la première phase du procédé à des températures entre 20°C et 80°C, et dans la seconde phase du procédé à des températures entre 50°C et 110°C.

5. Procédé selon la revendication 1, caractérisé en ce qu'on met en oeuvre, lors de la mise en réaction dans la première phase du procédé, par mole de triazolinethiones de formule (II) 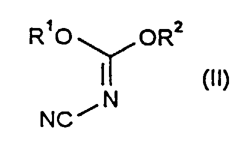 dans laquelle R¹ et R² ont les significations indiquées à la revendication 1, entre 3 et 4 moles d'un agent d'oxydation.

6. Procédé selon la revendication 1, caractérisé en ce qu'on effectue le procédé en mettant en oeuvre des adjuvants réactionnels appropriés.

7. Acides triazolsulfoniques, respectivement leurs sels répondant à la formule générale (III) 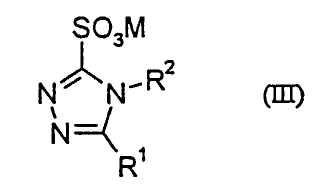 caractérisés en ce que
R¹ et R² ont la signification indiquée à la revendication 1, et
M représente un atome d'hydrogène ou un équivalent de métal alcalin ou de métal alcalino-terreux.

8. Acides triazolsulfoniques, respectivement leurs sels, répondant à la formule générale (III) selon la revendication 7, caractérisés en ce que
R¹ et R² ont les significations indiquées à la revendication 2 et
M représente un atome d'hydrogène, un atome de sodium ou un atome de potassium.
